# EUROPÄISCHE PATENTSCHRIFT

(11) **EP 2 303 198 B1**
(45) Veröffentlichungstag und Bekanntmachung des Hinweises auf die Patenterteilung: **07.10.2015**
(21) Anmeldenummer: 09777206.5
(22) Anmeldetag: 15.07.2009
(51) Int. Cl.: A61F 2/07, A61B 17/12, A61F 2/856, A61F 2/954, A61F 2/966, A61F 2/92

(54) **MEMBRANIMPLANTAT ZUR BEHANDLUNG VON HIRNARTERIENANEURYSMEN**
MEMBRANE IMPLANT FOR TREATMENT OF CEREBRAL ARTERY ANEURYSMS
IMPLANT À MEMBRANE DESTINÉ AU TRAITEMENT D'ANÉVRISMES CÉRÉBRAUX

(30) Priorität: 17.07.2008 DE 202008009604 U
(43) Veröffentlichungstag der Anmeldung: 06.04.2011
(73) Patentinhaber: Phenox GmbH, 44801 Bochum (DE); Sahl, Harald, 38304 Wolfenbüttel (DE)
(72) Erfinder: SAHL, Harald, 38304 Wolfenbüttel (DE)
(74) Vertreter: Schneiders, Josef
(86) Internationale Anmeldenummer: PCT/EP2009/005139
(87) Internationale Veröffentlichungsnummer: WO 2010/006777

(56) Entgegenhaltungen:
- EP-A1- 1 645 246
- WO-A1-2006/071245
- WO-A2-2006/111801
- WO-A2-2007/051179
- DE-A1- 10 301 600

## Beschreibung

Die Erfindung betrifft ein Membranimplantat zur Behandlung von Hirnarterienaneurysmen, wobei das Implantat in Gefäßsegmente an der Schädel- und Hirnbasis krankhafter (aneurysmatischer) Erweiterung endovaskulär implantierbar ist und in Kombination mit einem Stent das erkrankte Gefäßsegment von innen überbrückt und aus dem Blutstrom ausschaltet.

Die radiologisch-interventionelle Behandlung umschriebener krankhafter Erweiterungen von Schlagadern (Aneurysmen), durch endovaskuläre (d.h. durch die Blutbahn hindurch) Implantation blutundurchlässig beschichteter (gecoverter) Metallgitterprothesen (Stents), ist ausserhalb des Schädels gängige und erfolgreiche Praxis. Hierdurch wird der erkrankte Gefäßabschnitt überbrückt und ein weiteres Wachsen und letztlich eine Ruptur des Aneurysmas verhindert.

An den Gefäßen der Schädel- und Hirnbasis scheitert dieses Konzept bisher regelmäßig daran, dass verfügbare gecoverte Stents oder deren Einführbestecke nicht flexibel genug sind, um bis in die erkrankten Gefäßabschnitte vorgeführt werden zu können. Es gibt bisher lediglich unbeschichtete Stents, die über eine ausreichende Flexibilität auch des Einführbesteckes verfügen. Diese können auf Grund ihrer Blutdurchlässigkeit, Aneurysmen jedoch nicht aus der Zirkulation ausschalten.

WO-A-2006/111801 offenbart ein Membranimplantat mit den Merkmalen des Oberbegriffs aus Anspruch 1.

Entsprechend liegt der Erfindung das Problem zugrunde, ein endovaskulär platzierbares Implantat zu schaffen, das in Kombination mit bereits verfügbaren unbeschichteten Stents in der Lage ist, die Funktion eines bislang (und absehbar) nicht verfügbaren, geeigneten gecoverten Stents zur Behandlung von Hirnarterienaneurysmen zu übernehmen.

Dieses Problem wird mit einem Membranimplantat der eingangs beschriebenen Art gelöst, das eine biokompatible, zylindrische oder zylindersegmentförmige Kunststoffmembran aufweist und das Membranimplantat am proximalen Ende eine sich selbst aufrichtende Spange (2) aufweist, die die Zylinderform fixiert und gegen die Gefäßwandung abstützt.

Mit der Erfindung wird erreicht, dass der erkrankte Gefäßabschnitt mit dem Aneurysma von innen mit einer Membran überbrückt und aus dem Blutstrom ausgeschaltet wird. Das Membranimplantat wird in einem Mikrokatheter zusammengefaltet in den erkrankten Gefäßabschnitt vorgeführt. Nach korrekter Platzierung, wird das Implantat durch Rückzug des Mikrokatheters entfaltet und mit Hilfe eines Stent definitiv fixiert und stabilisiert, so dass die Funktionalität eines gecoverten Stent gegeben ist. Der Nachteil der ungenügenden Flexibilität wird durch sukzessive Implantation zweier flexibler Module umgangen.

Die erfindungsgemäßen Membranimplantate haben in der Regel eine Länge 5 mm und 30 mm und einen Radius zwischen 1 mm und 3 mm, wobei sich die Dimensionen aus der Dimension des erkrankten Gefäßsegments einerseits und der Art der Gefäßerweiterung andererseits ergeben. Die Folien haben eine Stärke im Bereich von 10 bis 50 µm; sie bestehen aus einem medizinisch verträglichen Kunststoff, beispielsweise aus Polyurethan, Polyalkylen, Polyamid, Polytetrafluoroethylen oder PET.

Die Kunststoffmembran des erfindungsgemäßen Implantats ist zylindrisch oder zylindersegmentförmig ausgebildet, wobei sich die Form aus dem expandierten Zustand im Gefäß nach der Implantation ergibt. Während der Implantation wird das Implantat in einem Mikrokatheter in mehr oder weniger zusammengefalteter Form geführt. Der Zylinder oder das Zylindersegment können geschlossen, aber auch offen, d. h. geschlitzt vorliegen. Letztere Ausführungsform hat den Vorteil, dass sich die Membran bei der Platzierung im zu behandelnden Gefäß optimal an den Gefäßdurchmesser anpassen kann, wobei sich die Seitenränder des Längsschlitzes geringfügig überlappen können oder auch einen Freiraum in Längsrichtung lassen.

Um eine optimale Anpassung an das Gefäß zu erzielen und das Implantat im implantierten Zustand unter Spannung zu halten, ist es sinnvoll, am proximalen und evtl. am distalen Ende eine sich selbst aufrichtende Spange vorzusehen, die die Zylinderform fixiert und gegen die Gefäßwandung abstützt. Eine solche Spannung kann beispielsweise aus einem Formgedächtnismaterial bestehen, etwa Nitinol. Die Spange kann beispielsweise auch aus einem röntgendichten Material bestehen, etwa Platin oder einer Platinlegierung, oder aus einem Formgedächtnismaterial, etwa Nitinol, das mit einer röntgendichten Ummantelung versehen ist. Eine solche Ummantelung kann beispielsweise in Form einer Drahtwendel aus einem röntgendichten Material wie Platin oder einer Platinlegierung vorliegen.

Anstelle von Drahtspangen können auch Kunststoffspangen vorgesehen sein, die die verlangten Formgedächtniseigenschaften aufweisen.

Die sich selbst aufrichtenden Spangen aus Draht oder Kunststoff können den Zylinder der Kunststoffmembran an einem oder beiden Enden vollständig oder teilweise einfassen. In der Regel ist eine Teileinfassung ausreichend. Dabei ist eine Einfassung des Membranzylinders am proximalen Ende, d. h. an dem Ende, das dem Führungsdraht bzw. Blutstrom zugewandt ist, vorgesehen.

Zusätzlich oder alternativ zur röntgendichten selbstaufrichtenden Spange kann ein röntgensichtbarer, flexibler Längsdraht vorgesehen sein, der entlang der Zylinderwand über die gesamt Zylinderlänge verläuft und ggf. an einem oder beiden Enden mit der sich selbst aufrichtenden Spange verbunden ist. Als bevorzugtes Material hierfür kann ein Draht aus Platin oder einer Platinlegierung dienen. Bekannt geworden für röntgensichtbare Implantate sind insbesondere Legierungen aus Platin und Iridium.

Zweckmäßigerweise ist das erfindungsgemäße Membranimplantat lösbar mit einem Führungsdraht verbunden, so dass es vom behandelnden Arzt zuverlässig platziert werden kann. Die Verbindung kann beispielsweise über eine Kupplungsstelle erfolgen, bei der Kupplungselement am Führungsdraht und an der proximalen oder distalen Spange vorgesehen sind. Ist die Spange aus einem Metall, kommen punktuelle Schweißverbindungen in Frage, die sich unter Stromeinwirkung in an und für sich bekannter Weise auflösen lassen. Die Technik ist die gleiche, wie sie für die Platzierung von Okklusionswendeln in Aneurysmen entwickelt wurde.

Gemäß einer bevorzugten Ausführungsform weist das Membranimplantat zwei Membranfolien auf, die übereinandergelegt werden. Dabei kann jede der Membranfolien beispielsweise eine Dicke von etwa 10 µm aufweisen. Etwaige Spangen, Drähte und sonstige Elemente können zwischen den beiden Membranfolien angeordnet sein, wobei eine Fixierung über Einschweißen oder Verkleben in Frage kommt.

In der Ausführungsform mit zwei Membranfolien können zwischen die Folien eingebrachte Verstärkungsfäden vorgesehen sein, beispielsweise solche aus Nitinol, die Formgedächtniseigenschaften aufweisen. Solche Drähte können in regelmäßigen Abständen angeordnet sein, beispielsweise solchen von 1 bis 10 mm, wobei die Abstände von der benötigen Spannkraft, der Stärke der Verstärkungsfäden und der Größe des Implantats abhängen. Vorzugsweise verlaufen diese Drähte schräg, beispielsweise in einem Winkel von 30 bis 60° und insbesondere von etwa 55° zur Längsrichtung des Implantats, wobei auch eine kreuzweise Anordnung der Drähte vorgesehen sein kann, so dass ein Netz- oder Gitterwerk entsteht. In die Formgedächtnisfäden können beispielsweise auch röntgendichte Filamente eingearbeitet sein, die eine bildliche Darstellung des Implantats im Körper ermöglichen.

Es versteht sich, dass die Verstärkungsfäden mit Formgedächtniseigenschaften auch beispielsweise aus einem geeigneten Kunststoff bestehen können.

Wie oben angesprochen, ist das erfindungsgemäße Implantat an einen Einführdraht gekoppelt und kann von diesem freigesetzt werden. Solche Einführdrähte sind an und für sich bekannt. Sie haben beispielsweise eine Durchmesser von 0,014 Inch (0,356 mm) und erlauben die Führung des Implantats durch einen konventionellen Mikrokatheter. Es ist zweckmäßig, dass ein solcher Führungsdraht durch das Lumen des zylinderförmigen Implantats geführt ist und an seinem distalen Ende zweckmäßigerweise röntgensichtbar ausgebildet ist. Der Führungsdraht kann an seinem distalen Ende oder in der Nähe des distalen Endes einen Kunststoffkonus aufweisen, der bei der Führung im Mikrokatheter und der Platzierung am erkrankten Gefäßsegment hilfreich ist.

Das Membranimplantat kann insbesondere aus einem vollständigen Zylinder bestehen, wird aber häufig auch aus einem Zylindersegment gebildet, das seitlich, einem Seitwandaneurysma gegenüberliegend, offen ist, so dass dort abgehende Gefäße nicht verschlossen werden. In diesem Fall hat das Implantat die Form eines schräg angeschnittenen Zylinders.

Die Erfindung betrifft schließlich ein Kit aus einem erfindungsgemäßen Membranimplantat zusammen mit einem darauf dimensionell abgestimmten Stent sowie den zur Führung von Stent und Implantat benötigten Führungsdrähten und ggf. Kathetern. In der Regel handelt es sich dabei um einen auf einen Ballon aufgekrimpten Stent, der durch hydraulische Expansion implantiert wird. In der Praxis kann auch ein Stent aus einem Formgedächtnismaterial, etwa Nitinol, verwandt werden, der nach Ausbringung aus einem Mikrokatheter an Ort und Stelle selbst expandiert und sich gegen Gefäßwandung unter Fixierung der Membran verspannt. Solche Stents sind an und für sich bekannt.

Die Erfindung wird anhand der beiliegenden Abbildungen näher erläutert. Es zeigen:
- Figur 1: einen Mikrokatheter mit (darin geführtem) zusammengefaltenten Implantat;
- Figur 2: das freigesetzte, entfaltete Implantat nach Figur 1 vor Ablösung des Führungsdrahtes;
- Figur 3: das freigesetzte, entfaltente Implantat gemäß Figur 1 nach Ablösung des Führungsdrahtes;
- Figur 4: das freigesetzte, entfaltete Implantat bei Behandlung von fusiformen Aneurysmas;
- Figur 5: ein freigesetztes, entfaltetes Implantat in Form eines Zylindersegments; und
- Figur 6: ein aus zwei Kunststoffmembranen bestehendes Implantat mit eingelegten Nitinolfilamenten.

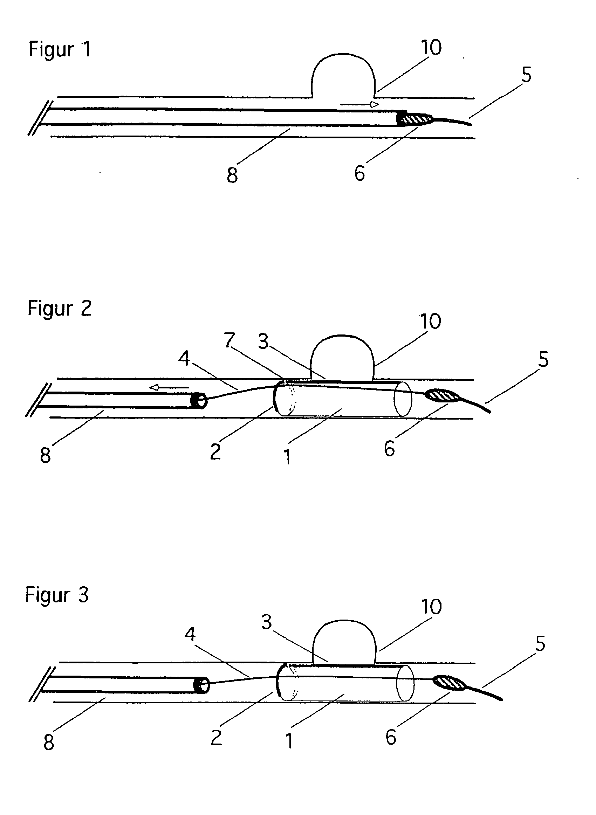

In den Abbildungen 1 bis 3 ist ein Ausführungsbeispiel der Erfindung zur Behandlung sogenannter Seitwandaneurysmen dargestellt.

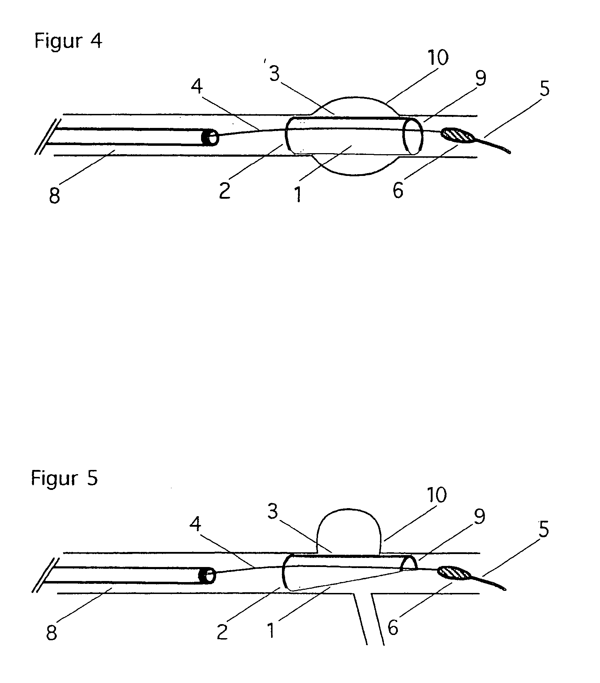

Abbildung 4 zeigt ein freigesetztes, entfaltetes Implantat nach Ablösung des Führungsdrahts, wie es zur Behandlung spindelförmiger (fusiformer) Aneurysmen eingesetzt werden kann.

Eine Ausgestaltung der Erfindung zur Behandlung von Seitwandaneurysmen, gegenüber denen ein Gefäß entspringt, dessen Abgang nicht verschlossen werden darf, ist in Abbildung 5 dargestellt. das Implantat besteht aus einer schräg angeschnittenen Membran, die proximal (dem Mikrokatheter bzw. behandelnden Arzt zugewandt) den vollem Umfang hat und distal (der dem behandelnden Arzt abgewandten Seite) auf einen Teilumfang verkürzt ist. Das entspringende Gefäß bleibt auf der offenen Seite des Membranimplantats 1 frei.

In den Figuren ist innerhalb des erkrankten Gefäßsegmentes 10 dargestellt:
das Membranimplantat mit Membran 1,
eine proximale selbst aufrichtende röntgensichtbare Drahtspange zum Entfalten und primären Fixieren der Membran 2,
ein flexibler röntgensichtbarer Längsdraht zum Stabilisieren und Markieren der Implantatlage 3,
ein ablösbarer Einführdraht 4 mit flexibler röntgensichtbarer Spitze 5 und Kunststoffkonus 6 am Austritt aus dem Mikrokatheter,
die Ablösestelle des Einführdrahtes 7,
sowie ein Mikrokatheter mit röntgensichtbar markierter Spitze zum Vorführen 8.

Zur Behandlung von Seitwandaneurysmen gegenüber denen ein Gefäß entspringt, dessen Abgang nicht verschlossen werden darf, sowie spindelförmiger (fusiformer) Aneurysmen, ist das Membranimplantat zwecks distaler Stabilisierung zusätzlich mit einer röntgensichtbaren, selbst aufrichtenden distalen Drahtspange 9 versehen.

Nach Rückzug des zum Vorführen an den Implantationsort benötigten Mikrokatheters, kann sich die Membran, unterstützt durch die selbst aufrichtende(n) Drahtspange(n) und den Blutstrom im erkrankten Gefäßsegment entfalten (Fig. 2). Unter Beobachtung insbesondere des röntgensichtbaren Längsdrahtes kann eine optimierte Platzierung hinsichtlich Längsposition und Rotation der Membran erreicht werden. Nach anschließender Ablösung des Einführdrahtes (Fig. 3) wird das jetzt nur durch den Druck des Blutes und die Drahtspange(n) labil fixierte Implantat durch einen (über den Einführdraht schon vor der Ablösung herangeführten) Stent, der das Membranimplantat proximal und distal überragen muss, endgültig in seiner Position fixiert.

Die Abbildungen 1 bis 3 zeigen eine erste Ausführungsform eines erfindungsgemäßen Membranimplantats in einem Blutgefäß mit einem Seitwandaneurysma 10. Das Implantat 1 hat im Wesentlichen zylindrische Formen und kann aus einem röhrenförmigen Kunststoffzylinder bestehen, wird jedoch in der Regel aus einem zu einem Zylinder verformten Kunststoffmembranblatt gebildet. Am proximalen Ende der Membran 1 befindet sich eine Metallspange 2, die vorzugsweise aus Nitinol besteht und der besseren Sichtbarkeit halber mit einem röntgendichten Material ummantelt sein kann. In Längsrichtung erstreckt sich ein flexibler röntgensichtbarer Längsdraht 3, der dem behandelnden Arzt die exakte Platzierung des Implantats in Bezug auf das Seitwandaneurysma 10 erlaubt.

Das Implantat wird über einen Mikrokatheter 8, der in der Regel, eine röntgendichte Spitze aufweist, an den Behandlungsort gebracht und aus diesem mit Hilfe eines Einführdrahts 4 freigesetzt. Der Einführdraht 4 ist über eine Schweiß-/Ablösstelle 7 mit der Spange 2 verbunden und kann durch kurze Einwirkung von Gleichstrom elektrolytisch abgelöst werden (siehe Abbildung 3). Der Führungsdraht selbst weist einen röntgensichtbare Spitze 5 zusammen mit einem Kunststoffball oder -konus 6 auf, die der Navigation und Abbildbarkeit dienen.

Die Kunststoffmembran weist die sich selbst aufrichtende Spange 2 vorzugsweise am proximalen Ende auf, um die Membran an die Gefäßwand anzulegen. Eine zweite Spange kann distal angeordnet sein, wie in Abbildung 4 dargestellt. In der Regel ist ausreichend, eine proximale Spange vorzusehen, die sich an der dem Blutstrom zugewandten Seite des Membranimplantats befindet. Bei fusiformen Aneurysmen oder Fehlbildungen, bei der es darauf ankommt, eine gute Kanalisierung des Blutstroms an der Fehlbildung vorbei zu gewährleisten, ist es allerdings zweckmäßig, eine zweite Spange 9 auch am distalen Ende des Membranimplantats 1 vorzusehen, siehe Abbildung 4.

Abbildung 5 zeigt den Spezialfall einer vaskulären Fehlbildung gegenüber einer Gefäßabzweigung. In diesem Fall muss das Membranimplantat die Abzweigung freilassen, weshalb es schräg angeschnitten ist. Auch in diesem Fall befinden sich am proximalen und am distalen Ende des Membranimplantats selbstaufrichtende Spangen 2 und 9.

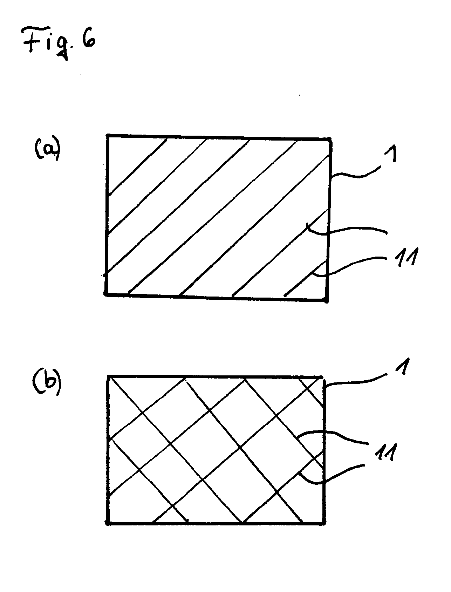

Figur 6 zeigt eine besondere Ausführungsform eines Membranimplantats, das zur Erzeugung einer höheren Spannung eingelagerter Nitinolfäden 11 aufweist. In diesem Fall besteht das Implantat aus einer Doppelmembran mit eingelagerten Nitinolfäden, die in einem regelmäßigen Abstand von beispielsweise 3 mm in einem Winkel von 45° zur Längsrichtung des Implantats verlaufen (Abbildung 6a). Die Verstärkungsfäden können auch ein Gitterwerk aufbilden, bei dem die Verstärkungsfäden 11 kreuz und quer rechtwinkelig zueinander verlaufen, ebenfalls in einem Winkel von 45° zur Längsrichtung des Implantats (Abbildung 6b). Das Implantat wird auf an und für sich bekannte Art und Weise mit einer Vorspannung versehen, in einem zusammengerollten Zustand in einen Mikrokatheter vorgeschoben und auf die gleiche Art und Weise freigesetzt, wie beispielsweise in den Abbildungen 1 bis 5 gezeigt. Der Führungsdraht kann dabei an eine der Verstärkungsdrähte 11 angeschweißt sein und wie bekannt elektrolytisch abgelöst werden. Bei der Freisetzung aus dem Mikrokatheter springt das Implantat nach Art einer Spiralfeder auf und legt sich an die Gefäßwand an, wobei die Fehlbildung, etwa ein Seitwandaneurysma, abgeschlossen wird.

Die Nitinolfäden können im Implantat zwischen den beiden Deckfolien eingeschweißt oder eingeklebt sein. Es ist möglich, einzelne Markierungsfäden aus einem röntgendichten Material einzuziehen, um auf diese Art und Weise das Membranimplantat röntgensichtbar zu machen.

Das erfindungsgemäße Membranimplantat wird in aller Regel durch einen anschließend eingebrachten Neurostent am Implantationsort zusätzlich fixiert und gesichert. Eine solche Sicherung kann allerdings bei der Ausführungsform der Abbildung 6 entbehrlich sein, da hier die Verstärkungsfäden mit einer ihnen aufgeprägten zylindrischen Form (Formgedächtnismaterial) die Sicherung im Gefäß übernimmt.

## Patentansprüche

1. Membranimplantat zur Behandlung von Hirnarterienaneurysmen (10), welches in Gefäßsegmente an der Schädel- und Hirnbasis mit krankhafter (aneurysmatischer) Erweiterung (10) endovaskulär implantierbar ist und wobei in Kombination mit einem Stent das erkrankte Gefäßsegment von innen überbrückt und aus dem Blutstrom ausgeschaltet wird, wobei das Membranimplantat eine biokompatible, zylindrische oder zylindersegmentförmige Kunststoffmembran (1) aufweist, **dadurch gekennzeichnet, dass** das Membranimplantat am proximalen Ende eine sich selbst aufrichtende Spange (2) aufweist, die die Zylinderform fixiert und gegen die Gefäßwandung abstützt.

2. Membranimplantat nach Anspruch 1, **dadurch gekennzeichnet, dass** das Membranimplantat entsprechend der Länge des zu behandelnden Gefäßsegments zwischen 5 mm und 30 mm lang ist und einen Radius entsprechend dem Radius des zu behandelnden Gefäßsegments zwischen 1 mm und 3 mm aufweist.

3. Membranimplantat nach Anspruch 1 oder 2, **dadurch gekennzeichnet, dass** die Kunststoffmembran (1) an ihrem distalen Ende eine selbstaufrichtende, röntgensichtbare Drahtspange (9) aufweist.

4. Membranimplantat nach einem vorstehenden Ansprüche, **dadurch gekennzeichnet, dass** die Kunststoffmembran (1) in der Längsachse des Zylinders einen röntgensichtbaren flexiblen Längsdraht (3) aufweist.

5. Membranimplantat nach einem der vorstehenden Ansprüche, **dadurch gekennzeichnet, dass** das Membranimplantat mit einem Führungsdraht (4) ablösbar verbunden ist, wobei der Führungsdraht (4) am Übergang zur Spitze (5) einen Kunststoffkonus (6) aufweist.

6. Membranimplantat nach Anspruch 5, **dadurch gekennzeichnet, dass** der Führungsdraht (4) eine flexible röntgensichtbare Spitze (5) aufweist.

7. Membranimplantat nach einem der vorstehenden Ansprüche, **dadurch gekennzeichnet, dass** die Kunststoffmembran (1) aus einem Zylindersegment besteht.

8. Membranimplantat nach einem der vorstehenden Ansprüche, **dadurch gekennzeichnet, dass** die Kunststoffmembran (1) längs geschlitzt ist.

9. Membranimplantat nach einem der vorstehenden Ansprüche, **dadurch gekennzeichnet, dass** das Membranimplantat zwei übereinandergelegte Membranfolien aufweist.

10. Membranimplantat nach Anspruch 9, **gekennzeichnet durch** zwischen die beiden Membranfolien eingebrachte Verstärkungsfäden (11).

11. Membranimplantat nach Anspruch 10, **dadurch gekennzeichnet, dass** die Verstärkungsfäden (11) aus Nitinol bestehen.

12. Membranimplantat nach Anspruch 10 oder 11, **dadurch gekennzeichnet, dass** der Abstand der Verstärkungsfäden (11) 1 bis 10 mm beträgt.

13. Membranimplantat nach einem der Ansprüche 10 bis 12, **dadurch gekennzeichnet, dass** die Verstärkungsfäden (11) schräg in einem Winkel von 30 bis 60° zur Längsrichtung des Membranimplantats verlaufen und ggf. kreuzweise angeordnet sind.

14. Membranimplantat nach einem der Ansprüche 9 bis 13, **gekennzeichnet durch** einen oder mehrere Verstärkungsfäden (11) in Form röntgensichtbarer, flexibler Drähte.

15. Kit, umfassend ein Membranimplantat nach einem der vorstehenden Ansprüche sowie einen Führungsdraht (4) und einen auf die Dimension des Membranimplantats abgestimmten Stent.

## Claims

1. Membrane implant (1) for treatment of cerebral artery aneurysms (10), wherein the implant can be implanted endovascularly into vessel segments (2) at the craniocerebral base of pathological (aneurysmatic) dilation (10) and, in combination with a stent, bridges the diseased vessel segment (2) from inside and disconnects it from the blood stream, wherein the membrane implant (1) comprises a biocompatible plastic membrane in the shape of a cylinder or a segment of a cylinder **characterized in that** the membrane (1) comprises a self-erecting clasp at its proximal end, which fixes the cylindrical shape and reinforces the cylindrical shape against the vessel wall.

2. Membrane implant according to claim 1, **characterized in that** the membrane implant (1) is between 5 mm and 30 mm long corresponding to the length of the vessel segment (2) to be treated and has a radius corresponding to the radius of the vessel segment to be treated, ranging between 1 mm and 3 mm.

3. Membrane implant according to claim 1 or 2, **characterized in that** the membrane (1) comprises a self-erecting X-ray visible wire clasp (9) at its proximal and/or distal end.

4. Membrane implant according to any one of the preceding claims, **characterized in that** the membrane (1) comprises an X-ray visible flexible elongated wire (3) in the longitudinal axis of the cylinder.

5. Membrane implant according to any one of the preceding claims, **characterized in that** the implant is detachably connected to a guiding wire (4) with a plastic cone (6) at the transition to the tip (5).

6. Membrane implant according to claim 5, **characterized in that** the guiding wire (4) comprises a flexible radiopaque tip (5).

7. Membrane implant according to any one of the preceding claims, **characterized in that** the membrane (1) is comprised of a cylinder segment.

8. Membrane implant according to any one of the preceding claims, **characterized in that** the membrane (1) is longitudinally slotted.

9. Membrane implant according to any one of the preceding claims, **characterized in that** it is comprised of two membrane foils.

10. Membrane implant according to claim 9, **characterized by** reinforcing filaments inserted between the two membrane foils.

11. Membrane implant according to claim 10, **characterized in that** the reinforcing filaments are made of nitinol.

12. Membrane implant according to claim 10 or 11, **characterized in that** the distance between the reinforcing filaments ranges between 1 and 10 mm.

13. Implant according to any one of the preceding claims 10 to 12, **characterized in that** the reinforcing filaments extend obliquely at an angle of 30 to 60° to the longitudinal direction of the membrane implant and that said filaments are possibly laid in crisscross arrangement.

14. Membrane implant according to any one of the preceding claims 9 to 13, **characterized by** one or more reinforcing filaments in the form of X-ray visible flexible wires.

15. Membrane implant according to any one of the preceding claims as a kit together with a guiding wire and a stent adapted to the dimension of the membrane implant.

## Revendications

1. Implant à membrane destiné au traitement des anévrismes cérébraux (10), lequel peut être implanté par voie endovasculaire dans des segments vasculaires (2) à la base du crâne et du cerveau au niveau d'une dilatation pathologique (anévrisme) (10) et permet, en association avec un stent, permet de ponter par l'intérieur le segment vasculaire atteint et de l'exclure de la circulation sanguine, ledit implant à membrane (1) comportant une membrane en plastique biocompatible, cylindrique ou en forme de segment de cylindre, **caractérisé en ce que** l'implant à membrane comporte, au niveau de l'extrémité proximale, une agrafe (2) qui se dresse par elle-même, fixe la forme cylindrique et prend appui contre la paroi vasculaire.

2. Implant à membrane selon la revendication 1, **caractérisé en ce que** l'implant à membrane, en fonction de la longueur du segment vasculaire à traiter, a une longueur entre 5 mm et 30 mm, et possède un rayon de 1 mm à 3 mm en fonction du rayon du segment vasculaire à traiter.

3. Implant à membrane selon la revendication 1 ou 2, **caractérisé en ce que** la membrane plastique (1) comporte, au niveau de son extrémité distale, une agrafe (9) en fil métallique se dressant par elle-même et visible aux rayons X.

4. Implant à membrane selon l'une quelconque des revendications précédentes, **caractérisé en ce que** la membrane plastique (1) comporte, dans l'axe longitudinal du cylindre, un fil métallique longitudinal (3) flexible visible aux rayons X.

5. Implant à membrane selon l'une quelconque des revendications précédentes, **caractérisé en ce que** l'implant à membrane est relié de manière amovible à un fil métallique de guidage (4), ledit fil métallique de guidage (4) comportant un cône plastique (6) au niveau de la transition vers la pointe (5).

6. Implant à membrane selon la revendication 5, **caractérisé en ce que** le fil métallique de guidage (4) comporte une pointe (5) flexible, visible aux rayons X.

7. Implant à membrane selon l'une quelconque des revendications précédentes, **caractérisé en ce que** la membrane plastique (1) est formée par un segment de cylindre.

8. Implant à membrane selon l'une quelconque des revendications précédentes, **caractérisé en ce que** la membrane plastique (1) est fendue dans le sens de la longueur.

9. Implant à membrane selon l'une quelconque des revendications précédentes, **caractérisé en ce que** l'implant à membrane comporte deux feuilles formant membrane, posées l'une sur l'autre.

10. Implant à membrane selon la revendication 9, **caractérisé par** des fils de renfort (11) insérés entre les deux feuilles formant membrane.

11. Implant à membrane selon la revendication 10, **caractérisé en ce que** les fils de renfort (11) sont réalisés en nitinol.

12. Implant à membrane selon la revendication 10 ou 11, **caractérisé en ce que** la distance entre les fils de renfort (11) est de 1 à 10 mm.

13. Implant à membrane selon l'une quelconque des revendications 10 à 12, **caractérisé en ce que** les fils de renfort (11) s'étendent en oblique en formant un angle de 30 à 60° avec la direction longitudinale de l'implant à membrane et, le cas échéant, sont disposés en croix.

14. Implant à membrane selon l'une quelconque des revendications 9 à 13, **caractérisé par** un ou plusieurs fils de renfort (11) en forme de fils métalliques flexibles, visibles aux rayons X.

15. Kit, comportant un implant à membrane selon l'une des revendications précédentes, ainsi qu'un fil métallique de guidage (4) et un stent ajusté à la dimension de l'implant à membrane.
